(19) Europäisches Patentamt
European Patent Office
Office européen des brevets

(11) Veröffentlichungsnummer : **0 048 695**
**B1**

(12) # EUROPÄISCHE PATENTSCHRIFT

(45) Veröffentlichungstag der Patentschrift : **13.06.84**

(51) Int. Cl.³ : **C 07 D457/12**, C 07 D457/04, C 07 D457/06, A 61 K 31/48

(21) Anmeldenummer : **81810382.2**

(22) Anmeldetag : **17.09.81**

(54) **Verfahren zur Isomerisierung von Ergolinderivaten.**

(30) Priorität : **23.09.80 CH 7122/80**

(43) Veröffentlichungstag der Anmeldung : **31.03.82 Patentblatt 82/13**

(45) Bekanntmachung des Hinweises auf die Patenterteilung : **13.06.84 Patentblatt 84/24**

(84) Benannte Vertragsstaaten : **AT BE CH DE FR GB IT LI LU NL SE**

(56) Entgegenhaltungen :
**EP-A- 0 021 206**
**EP-A- 0 032 684**
**CH-A- 585 750**
**DE-A- 2 530 577**
**DE-A- 2 656 344**
**US-A- 4 246 265**
**M. FIESER & L.F. FIESER, "Reagents for organic synthesis", Band 4, 1974, Seiten 301-311, Wiley-Interscience Publication, New York, U.S.A.**
**M. FIESER & L.F. FIESER, "Reagents for organic synthesis", Band 8, 1974, Seiten 294-295, Wiley-Interscience Publication New York, U.S.A.**

(73) Patentinhaber : **SANDOZ AG**
**Lichtstrasse 35**
**CH-4002 Basel (CH)**

(72) Erfinder : **Brich, Zdenek, Dr.**
**Holeeholzweg 63**
**CH-4102 Binningen (CH)**
Erfinder : **Mühle, Herbert, Dr.**
**Binzenweg 6C**
**CH-4102 Binningen (CH)**

**EP 0 048 695 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Isomerisierung von in 8β-Stellung substituierten Ergolinen, zu den entsprechenden 8α-Verbindungen.

Die 8α-substituierten Ergoline entsprechen einer unnatürlichen Serie der Mutterkornalkaloide und können aus den natürlichen 8β-substituierten Ergolinderivaten hergestellt werden.

Ein wesentliches 8α-Ergolin ist der iso-9,10-Dihydrolysergsäuremethylester. Diese Verbindung wurde bis jetzt hergestellt, indem man 9,10-Dihydrolysergsäuremethylester in das 8,9- oder 7,8-Ergolen überführte, das anschliessend zu einem Gemisch enthaltend iso-9,10-Dihydrolysergsäuremethylester hydriert wurde. Dieses mehrstufige Verfahren führt mit unbefriedigenden Ausbeuten zu einer oft unreinen Verbindung. Es wurde auch versucht, den 9,10-Dihydrolysergsäuremethylester unter equilibrierenden d. h. schwach basischen Bedingungen mit Natriummethanolat in Methanol zu epimerisieren, wobei aber vorwiegend das β-Isomer erhalten wurde, da das α-Isomer thermodynamisch weniger stabil ist.

Es wurde nun überraschenderweise gefunden, dass eine unter kinetischen Bedingungen durchgeführte Protonierung eines durch Entfernen des 8α-Protons erhaltenen Anions eines 8β-substituierten Ergolins überwiegend das 8α-Isomere liefert. Erfindungsgemäss werden 8α-substituierte Ergoline hergestellt, indem man in einer ersten Stufe ein Anion eines in 8β-Stellung durch eine elektronenanziehende Gruppe substituierten Ergolins durch Entfernen eines in 8α-Stellung stehenden Protons bildet und in einer zweiten, getrennt durchgeführten Stufe das erhaltene Anion protoniert, und nötigenfalls das erhaltene 8α-Isomere in das gewünschte Ergolin überführt. Die zur erfindungsgemässen Isomerisierung geeigneten Ergoinderivate umfassen sowohl die in der Natur vorkommenden wie auch die synthetisch zugänglichen in 8β-Stellung durch eine elektronenanziehende Gruppe substituierten Ergolinderivate. Sie können z. B. die in der Chemie der Lysergsäurederivate üblichen Substituenten tragen. Das Stickstoffatom in Stellung 1 ist bevorzugt unsubstituiert. Als in 8β-Stellung stehende, elektronenanziehende Gruppe kommen alle üblichen Radikale in Frage, die das Entfernen des in 8α-Stellung stehenden Protons, z. B. durch Bildung eines Enolats, ermöglichen, beispielsweise das funktionelle Radikal eines Esters, Thioesters, Amids, Aldehyds, Hydrazids, Imins, Hydrazons, Nitrils oder Ketons, vorzugsweise eines Esters oder Amids. Diese Reste können aliphatische oder aromatische Gruppen enthalten. Steht der elektronenanziehende Rest für das funktionelle Radikal eines Esters, so entspricht er der Formel —COOR, worin R für einen aliphatischen oder aromatischen Kohlenwasserstoffrest steht, insbesondere für niederes Alkyl, beispielsweise für Methyl. Die anderen, oben erwähnten funktionellen Radikale entsprechen z. B. den folgenden Gruppen : Alkylthiocarbonyl, Arylthiocarbonyl, Formyl, Cyano, Acyl, gegebenenfalls durch Alkyl oder Aryl substituiertes Carbamoyl, Hydrazinocarbonyl, Iminomethyl oder Hydrazonomethyl.

Die zur erfindungsgemässen Isomerisierung geeigneten Ergolinderivate umfassen insbesondere 9, 10-Dihydrolysergsäurederivate, beispielsweise die Verbindungen der Formel I,

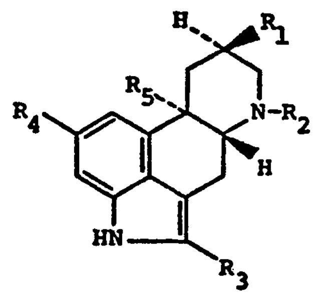

(I)

worin

$R_1$ einen elektronenanziehenden Rest bedeutet,
$R_2$ für Alkyl mit 1 bis 4 Kohlenstoffatomen steht,
$R_3$ für Wasserstoff, Methyl oder Halogen mit einer Atomzahl von 9 bis 35 steht,
$R_4$ Wasserstoff oder Brom bedeutet und
$R_5$ für Wasserstoff oder Methoxy steht.

Die Ueberführung des 8β-Isomeres in das entsprechende Anion kann auf an sich bekannte Weise unter stark basischen Bedingungen, z. B. durch Einwirkung einer unter den Reaktionsbedingungen starken, nicht nukleophilen Base durchgeführt werden. Es wird zweckmässigerweise ein Lithium-Enolat hergestellt. Als Base verwendet man beispielsweise Lithium-dicyclohexylamid, Lithium-tetramethylpiperidid, Lithiumisopropylcyclohexylamid oder vorzugsweise Lithiumdiisopropylamid, das *in situ* hergestellt werden kann. Bevorzugt wird mindestens ein Aequivalent Base pro zu entfernendem Proton eingesetzt. Da im Falle eines in 1-Stellung unsubstituierten Ergolins das entsprechende Proton primär entfernt wird, werden vorzugsweise zur Bildung des Dianions mindestens 2 Aequivalente Base bezogen auf das Ergolin eingesetzt. Falls das Ergolin in 6-Stellung ebenfalls unsubstituiert ist, werden 3 Aequivalente benötigt. Es wird vorzugsweise nicht mehr als ein Aequivalent Ueberschuss verwendet.

Die Bildung des Anions kann in einem aprotischen, polaren Lösungsmittel, beispielsweise in einem Aether wie Tetrahydrofuran durchgeführt werden. Die Reaktionstemperatur liegt zweckmässigerweise zwischen − 110 und + 25 °C, vorzugsweise zwischen − 40 und − 10 °C.

Die anschliessende Protonierung des Anions kann auf an sich bekannte Weise durch Zugabe einer Protonquelle durchgeführt werden. Als Protonquelle verwendet man beispielsweise Wasser oder Methanol. Man kann auch eine Säure verwenden, wie z. B. Essigsäure, Salzsäure oder Weinsäure, zweckmässigerweise in wässrigem Medium oder auch in einem organischen Lösungsmittel, z. B. in einem Aether wie Tetrahydrofuran. Die Protonierung erfolgt vorzugsweise bei einer niederen Temperatur, beispielsweise unter − 20 °C.

Das erfindungsgemässe Verfahren liefert besonders im Falle von in Stellung 1 unsubstituierten Ergolinen einen überraschend grossen Anteil an 8α-substituierten Ergolinderivaten, neben einem geringen Anteil der entsprechenden 8β-substituierten Verbindungen. Das 8α-Isomere kann nach an sich bekannten Methoden getrennt werden, z. B. durch Kristallisation. Gewünschtenfalls kann der Anteil des 8β-substituierten Ergolins reduziert werden, indem man dieses Isomere in eine weitere Verbindung überführt, falls es schneller reagiert als das 8α-Isomere. So kann man zum Beispiel ein Gemisch von 9,10-Dihydrolysergsäuremethylester und iso-9,10-Dihydrolysergsäuremethylester einer stoechiometrisch gesteuerten partiellen Verseifung in Anwesenheit eines Alkalimetall-Alkoholats unterwerfer- und das erhaltene Salz des 8β-Isomeres vom Ester des 8α-Isomeres, das langsamer verseift, auf an sich bekannte Weise trennen.

Die erfindungsgemäss hergestellten 8α-substituierten Ergoline sind im allgemeinen bekannte, pharmakologisch aktive Verbindungen oder wertvolle Zwischenprodukte, die als Ausgangsverbindungen zur Herstellung von therapeutisch aktiven 8α-Ergolinderivaten, beispielsweise von 8α-Sulfamoylaminoergolinen und anderen 8α-Aminoergolinen, geeignet sind. So kann zum Beispiel ein erfindungsgemäss hergestelltes, in 8α-Stellung durch einen funktionellen Radikal eines Esters substituiertes Ergolinderivat in ein entsprechendes 8α-Sulfamoylamino-ergolinderivat übergeführt werden, indem man den Ester z. B. mit einem Gemisch von Hydrazinhydrat und Hydrazindihydrochlorid umsetzt, das entstandene Hydrazid nach Curtius in das entsprechende 8α-Aminoderivat umwandelt und letzteres nach an sich bekannten Methoden, z. B. wie in der DOS Nr. 2656344 beschrieben, mit einem reaktiven Derivat der entsprechenden Sulfaminsäure umsetzt.

Aus einem erfindungsgemäss hergestellten, in 8α-Stellung durch einen funktionellen Radikal eines Amids, Aldehyds, Nitrils oder Imins substituiertem Ergolinderivat kann man nach an sich bekannten Methoden über die freie 8α-Carbonsäure z. B. den Methylester herstellen und letzteren wie oben angegeben in das gewünschte 8α-Sulfamoylaminoderivat überführen.

Es können beispielsweise 8α-Sulfamoylaminoderivate der Formel II hergestellt werden,

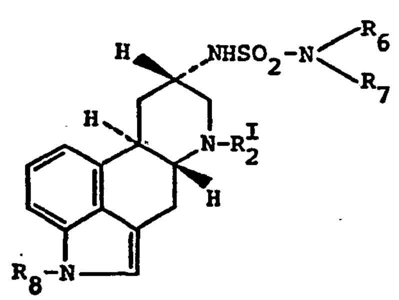

(II)

worin

$R_2^1$ für Alkyl mit 1 bis 4 Kohlenstoffatomen steht,

$R_6$ Wasserstoff oder Alkyl mit 1 bis 3 Kohlenstoffatomen bedeutet,

$R_7$ für Alkyl mit 1 bis 4 Kohlenstoffatomen steht und

$R_8$ Wasserstoff oder Methyl bedeutet.

Solche Verbindungen sind aus den DOS Nrn 2656344 und 2530577 bekannt, wie z. B. das N-(1,6-Dimethylergolin-8α-yl)-N',N'-dimethylsulfamid und das N-(6-Methylergolin-8α-yl)-N',N'-diäthylsulfamid.

Als Verbindungen der Formel II können ferner das N,N-Diäthyl-N'-(6-n-propylergolin-8α-yl)sulfamid, das N,N-Diäthyl-N'-(1-methyl-6-n-propylergolin-8α-yl) sulfamid und das N-(6-Aethylergolin-8α-yl)-N',N'-dimethylsulfamid genannt werden. Diese Verbindungen sind in der deutschen Patentanmeldung P 3127845.0 und in äquivalenten europäischen Anmeldungen beschrieben.

Es können auch N-Ergolinyl-N',N'-diäthylharnstoffderivate der Formel III hergestellt werden,

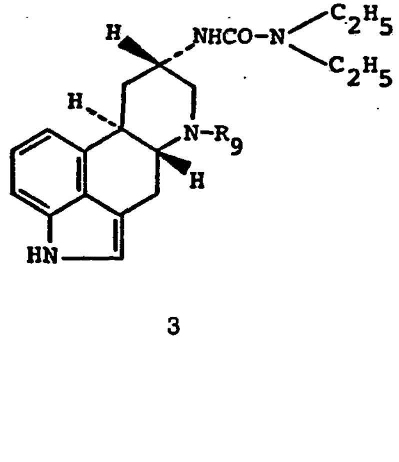

(III)

worin $R_9$ für alkyl mit 1 bis 6 Kohlenstoffatomen,
$-(CH_2)n-CH = CH_2$, $-(CH_2)_n-C \equiv CH$, $-(CH_2)_n-COOR'$,
$-(CH_2)_n-CN$ oder $-(CH_2)_m-\underline{CH-(CH_2)_n-CH_2}$ steht,

wobei n 1 oder 2, m 0 der 1 bedeutet und R' für Alkyl mit 1 bis 6 Kohlenstoffatomen steht. Diese Verbindungen sind in der europäischen Patentanmeldung Nr. 21206 beschrieben.

Als Verbindungen der Formel III können N-(6-n-Propylergolin-8α-yl)-N',N'-diäthylharnstoff und N-(6-Methylergolin-8α-yl)-N',N'-diäthylharnstoff genannt werden.

Die Verbindungen der Formeln II und III weisen im allgemeinen dopaminerge und Prolaktinsekretionshemmende Eigenschaften auf.

Das nachfolgende Beispiel erläutert die Erfindung. Temperaturangaben erfolgen in Celsiusgraden und sind unkorrigiert.

Beispiel

Iso-9,10-Dihydrolysergsäuremethylester

Eine Lösung von 72,1 g (0,71 Mol) Diisopropylamin in 750 ml Tetrahydrofuran wird unter Argon auf − 30° gekühlt und mit 308,3 ml (0,66 Mol) n-Butyllithium (2,14 M in Cyclohexan) versetzt. Die entstandene Lösung wird während 30 Minuten nachgerührt. Dann tropft man eine Lösung von 85,2 g (0,3 Mol) 9,10-Dihydrolysergsäure-methylester in 750 ml Tetrahydrofuran zu. Anschliessend rührt man 30 Minuten bei − 35° nach. Die Hydrolyse erfolgt durch Zugabe von 384 ml Salzsäure 10 %-ig (1,1 Mol) bei − 30°. Dann wird auf Raumtemperatur erwärmt. Die organische Phase wird abgetrennt, mit Magnesiumsulfat getrocknet und eingedampft. Man erhält 84,3 g (99 %) Eindampfrückstand, der praktisch ausschliesslich die beiden möglichen Stereoisomeren im Verhältnis 85 : 15 enthält. Das Rohprodukt wird zur Reinigung aus Methylenchlorid/Hexan (1 : 2) umkristallisiert. Man erhält in 75 % Ausbeute praktisch einheitliches Iso-9,10-Dihydrolysergsäuremethylester. Smp.: 186,9-187,4 °C. Die physikalischen Eigenschaften, spektroskopischen Daten und Mikroanalyse stimmen mit den Daten von auf andere Weise erhaltenem Iso-9,10-Dihydrolysergsäuremethylester überein.

Die erhaltene Verbindung kann mittels Hydrazinhydrat/Hydrazin-dihydrochlorid in Propanol in das entsprechende Hydrazid übergeführt werden. Das Hydrazid kann mit Natriumnitrit in wässriger Salzsäure behandelt, auf 80° erwärmt und zum 8α-Amino-6-methylergolin aufgearbeitet werden. Diese Verbindung kann gegebenenfalls in 1-Stellung methyliert werden und mit Dimethyl- oder Diäthyl-chlorsulfamid acyliert werden, wobei z. B. das N-(1,6-dimethylergolin-8α-yl)-N',N'-dimethylsulfamid oder das N-(6-methylergolin-8α-yl)-N',N'-diäthylsulfamid erhalten werden.

**Ansprüche**

1. Verfahren zur Herstellung eines in 8α-Stellung durch eine elektronenanziehende Gruppe substituierten Ergolins, dadurch gekennzeichnet, dass man in einer ersten Stufe ein Anion eines in 8β-Stellung durch eine elektronenanziehende Gruppe substituierten Ergolins durch Entfernen eines in 8α-Stellung stehenden Protons bildet und in einer zweiten, getrennt durchgeführten Stufe das so erhaltene Anion protoniert.

2. Verfahren gemäss Anspruch 1, dadurch gekennzeichnet, dass man ein in 1-Stellung unsubstituiertes Ergolin deprotoniert.

3. Verfahren gemäss Anspruch 2, dadurch gekennzeichnet, dass man Verbindungen der Formel I deprotoniert,

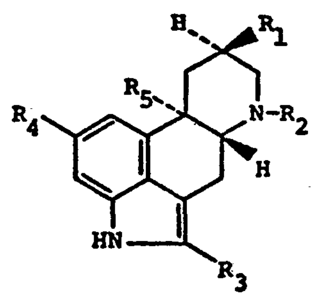

(I)

worin
$R_1$ eine elektronenanziehende Gruppe bedeutet,
$R_2$ für Alkyl mit 1 bis 4 Kohlenstoffatomen steht,
$R_3$ für Wasserstoff, Methyl oder Halogen mit einer Atomzahl von 9 bis 35 steht,
$R_4$ Wasserstoff oder Brom bedeutet und
$R_5$ für Wasserstoff oder Methoxy steht.

4. Verfahren gemäss einem der Ansprüche 1 bis 3, dadurch gekennzeichnet, dass man Ergoline deprotoniert, die in 8β-Stellung eine Ester-, Amid-, Aldehyd-, Hydrazid-, Imin-, Hydrazon- oder Keton-Gruppe aufweisen.

5. Verfahren gemäss einem der Ansprüche 1 bis 4, dadurch gekennzeichnet, dass die Deprotonierung durch Einwirkung einer unter den Reaktionsbedingungen starken, nicht nukleophilen Base durchgeführt wird.

6. Verfahren gemäss einem der Ansprüche 1 bis 5, dadurch gekennzeichnet, dass das erhaltene Anion ein Lithium-Enolat ist.

7. Verfahren zur Herstellung eines in 8α-Stellung substituierten Ergolins, dadurch gekennzeichnet, dass ein in 8α-Stellung durch eine elektronenanziehende Gruppe substituiertes Ergolin nach einem Verfahren gemäss einem der Ansprüche 1 bis 6 hergestellt wird und dann in ein weiteres in 8α-Stellung substituiertes Ergolin überführt wird.

8. Verfahren gemäss Anspruch 7, dadurch gekennzeichnet, dass man das in 8α-Stellung durch eine elektronenanziehende Gruppe substituierte Ergolin in ein 8α-(gegebenenfalls substituiertes amino)ergolin überführt.

9. Verfahren gemäss Anspruch 7, dadurch gekennzeichnet, dass man das in 8α-Stellung durch eine elektronenanziehende Gruppe substituierte Ergolin in ein 8α-Sulfamoylamino-ergolin überführt.

10. Verfahren gemäss Anspruch 7, dadurch gekennzeichnet, dass man das in 8α-Stellung durch eine elektronenanziehende Gruppe substituierte Ergolin in das N-(1,6-Dimethylergolin-8α-yl)-N',N'-dimethylsulfamid überführt.

11. Verfahren gemäss Anspruch 7, dadurch gekennzeichnet, dass man das in 8α-Stellung durch eine elektronenanziehende Gruppe substituierte Ergolin in das N-(6-Methylergolin-8α-yl)-N',N'-diäthylsulfamid, das N,N-Diäthyl-N'-(1-methyl-6-n-propylergolin-8α-yl)sulfamid, das N-(6-n-Propylergolin-8α-yl)-N', N'-diäthylharnstoff oder das N-(6-Methylergolin-8α-yl)-N',N'-diäthylharnstoff überführt.

**Claims**

1. Process for the production of an ergoline substituted in the 8α-position by an electron attracting group characterised in that in a first step an anion of an ergoline substituted in the 8β-position by an electron attracting group is formed by removal of a proton in the 8α-position and in a second separately carried out step the so obtained anion is protonated.

2. Process according to claim 1, characterised in that an ergoline unsubstituted in the 1 position is deprotonated.

3. Process according to claim 2, characterised in that compounds of formula I

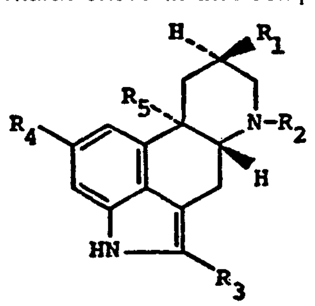

(I)

wherein

$R_1$ is an electron attracting group,
$R_2$ is alkyl of 1 to 4 carbon atoms,
$R_3$ is hydrogen, methyl or halogen of an atomic number of from 9 to 35,
$R_4$ is hydrogen or bromine, and
$R_5$ is hydrogen or methoxy are deprotonated.

4. Process according to any one of claims 1 to 3, characterised in that ergolines having in the 8β position an ester, amide, aldehyde, hydrazide, imine, hydrazone, or ketone group, are deprotonated.

5. Process according to any one of claims 1 to 4, characterised in that the deprotonation is carried out by a base which is strong and non-nuceophilic under the reaction conditions.

6. Process according to any one of claims 1 to 5, characterised in that the so obtained anion is a lithium enolate.

7. Process for the production of an ergoline substituted in the 8α position characterised in that in a first step an ergoline substituted in the 8α-position by an electron attracting group is produced according to a process according to any one of claims 1 to 6 and in a further step is converted into an ergoline substituted in the 8α position.

8. Process according to claim 7, characterised in that an ergoline substituted in the 8α-position by an electron attracting group is converted into an 8α-(optionally substituted amino)ergoline.

9. Process according to claim 7, characterised in that an ergoline substituted in the 8α-position by an electron attracting group is converted into a 8α-sulfamoylamino ergoline.

10. Process according to claim 7, characterised in that an ergoline substituted in the 8α-position by an electron attracting group is converted into N-(1,6-dimethylergoline-8α-yl)-N',N'-dimethylsulfamide.

11. Process according to claim 7, characterised in that an ergoline substituted in the 8α-position by an electron attracting group is converted into N-(6-methylergolin-8α-yl)-N',N'-diethylsulfamide, N,N-diethyl-N'-1(methyl-6-n-propylergoline-8α-yl)sulfamide, N-(6-n-propylergoline-8α-yl)-N',N'-diethylurea or N-(6-methylergoline-8α-yl)-N',N'-diethylurea.

**Revendications**

1. Procédé de préparation d'une ergoline substituée en position 8α par un groupe attracteur d'électrons, caractérisé en ce qu'on forme dans une première étape un anion d'une ergoline substituée en position 8β par un groupe attracteur d'électrons par élimination d'un proton situé en position 8α et on protone l'anion ainsi obtenu dans une deuxième étape, effectuée séparément.

2. Procédé selon la revendication 1, caractérisé en ce qu'on déprotone une ergoline non substituée en position 1.

3. Procédé selon la revendication 2, caractérisé en ce qu'on déprotone des composés de formule I

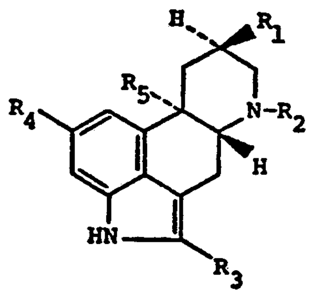

(I)

dans laquelle

$R_1$ signifie un groupe attracteur d'électrons,

$R_2$ représente alkyle contenant de 1 à 4 atomes de carbone,

$R_3$ représente l'hydrogène, méthyle ou un halogène ayant un nombre atomique de 9 à 35,

$R_4$ signifie l'hydrogène ou le brome et

$R_5$ représente l'hydrogène ou méthoxy.

4. Procédé selon une des revendications 1 à 3, caractérisé en ce qu'on déprotone des ergolines qui présentent en position 8β un groupe ester, amide, aldéhyde, hydrazide, imine, hydrazone ou cétone.

5. Procédé selon une des revendications 1 à 4, caractérisé en ce que la déprotonation est effectuée par action d'une base forte, non nucléophile, sous les conditions de réaction.

6. Procédé selon une des revendications 1 à 5, caractérisé en ce que l'anion obtenu est un énolate de lithium.

7. Procédé de préparation d'une ergoline substituée en position 8α, caractérisé en ce que une ergoline substituée en position 8α par un groupe attracteur d'électrons est préparée d'après un procédé selon une des revendications 1 à 6 et est ensuite transformée en une autre ergoline substituée en position 8α.

8. Procédé selon la revendication 7, caractérisé en ce qu'on transforme l'ergoline substituée en position 8α par un groupe attracteur d'électrons en une 8α-(amino éventuellement substitué)ergoline.

9. Procédé selon la revendication 7, caractérisé en ce qu'on transforme l'ergoline substituée en position 8α par un groupe attracteur d'électrons en 8α-sulfamoylamino-ergoline.

10. Procédé selon la revendication 7, caractérisé en ce qu'on transforme l'ergoline substituée en position 8α par un groupe attracteur d'électrons en N-(1,6-diméthylergoline-8α-yl)-N',N'-diméthylsulfamide.

11. Procédé selon la revendication 7, caractérisé en ce qu'on transforme l'ergoline substituée en position 8α par un groupe attracteur d'électrons en N-(6-méthylergoline-8α-yl)-N',N'-diéthylsulfamide, en N,N-diéthyl-N'-(1-méthyl-6-n-propylergoline-8α-yl) sulfamide, en N-(6-n-propylergoline-8α-yl)-N',N'-diéthylurée ou en N-(6-méthylergoline-8α-yl)-N',N'-diéthylurée.